# EUROPEAN PATENT APPLICATION

(11) **EP 3 047 825 A1**
(43) Date of publication of application: **27.07.2016**
(21) Application number: 15152326.3
(22) Date of filing: 23.01.2015
(51) Int. Cl.: A61F 9/08, A61H 3/06

(54) **Directional guidance apparatus**

(71) Applicant: ADI Access Limited, Helston, Cornwall TR12 7LD (GB)
(72) Inventor: Kemp, Helen, Helston, Cornwall TR12 7LD (GB)
(74) Representative: Bryers LLP

(57) **Abstract**

The present invention relates to a directional guidance apparatus, comprising: at least one detector (8) operable to detect a user within and/or entering a predetermined region; a storage device operable to store an audio output comprising at least one pre-recorded directional and/or instructional audio message; at least one speaker (10) in communication with the audio output of the storage device; and an audio controller operable to control the audio output to the speaker on detection of a user within the predetermined region in order to provide at least one audio message.

## Description

### TECHNICAL FIELD

The present invention relates to a directional guidance apparatus, and a method, for providing directional instructions to a user within a predetermined region.

### BACKGROUND

Visually impaired people find it difficult to find their way around and to operate equipment in unfamiliar surroundings, such as for example unfamiliar bathrooms/toilets. As a result, visually impaired people require the assistance of another person to describe the layout of the room and equipment before being able to use the facilities.

The location of sanitary ware within bathroom/toilet facilities, the dimensions of the area and the door locks can vary greatly which can cause a lot of concern and stress to the visually impaired user. There is therefore a need for an apparatus and method which can provide directional guidance to a visually impaired user such that the user can use the facilities independently and with confidence.

### SUMMARY OF THE INVENTION

According to a first aspect, the present invention provides a directional guidance apparatus, comprising at least one detector operable to detect a user within and/or entering a predetermined region, a storage device operable to store an audio output comprising at least one pre-recorded directional and/or instructional audio message, at least one speaker in communication with the audio output of the storage device, and an audio controller operable to control the audio output to the speaker on detection by the at least one detector of a user within the predetermined region in order to provide at least one audio message.

The apparatus may comprise a main body unit comprising a mountable portion comprising a mounting surface for mounting the main body unit on a surface of or within the predetermined region, such as for example on a wall, ceiling of or an appliance within the predetermined region. The main body unit may comprise the storage device, the at least one speaker, the audio controller. The apparatus may further comprise a detector portion comprising a detector surface comprising the at least one detector.

The detector portion and the mountable portion may be provided within an integral main body unit. The plane defined by the detector surface of the detector portion may extend substantially parallel to the mounting surface of the mounting portion. The plane defined by the detector surface may extend at a predetermined angle, for example at a predetermined acute angle, to mounting surface of the mounting portion. The detector portion may be moveable relative to the mountable portion so as to adjust the angle of the detector surface relative to the mounting surface.

The detector portion may be provided by a separate unit to the main body unit. The detector portion may comprise a detector mounting portion comprising a surface for mounting the detector portion on a surface of or within a predetermined region, such as for example on a wall or ceiling of or on an appliance within the predetermined region.

The apparatus may comprise a portable unit in communication with at least one of the detector(s), the speaker(s), the audio controller, and the storage device. The portable unit is configured to be carried by the user, such as for example by hand or on a lanyard.

For example, the portable unit may comprise at least one speaker, in which the portable unit, in particular the at least one speaker of the portable unit, is in communication with the storage device and the audio controller.

The portable unit may be in communication with the detector(s). For example, the portable unit may be operable to emit communication signals using any suitable transport medium. The detector(s) may be operable to detect communication signals, such as for example radiofrequency identification tags (RFED tag) or short range radiofrequency signals (including, but not limited to, Bluetooth and zigbee signals), emitted from the portable unit. The signals emitted from the portable unit may advantageously include a form of identification which can therefore help to prevent unauthorised access and/or use of the predetermined region.

The term "portable" is herein used to refer to any unit which is not physically connected (by for example wire, fibre-optics etc) to the audio controller, and is operable to receive signals from the audio controller by one or more suitable transport mediums.

The portable unit preferably comprises a module operable to receive signals from the audio controller to control the audio output of the speaker.

At least one of the storage device, the audio controller and the detector(s) may be provided as a separate unit to the portable unit. For example, the storage device and the audio controller, and optionally the at least one detector, may be provided within a main body unit mountable to a surface of the predetermined region.

The storage device and the audio controller may be provided within the portable unit of the apparatus.

The apparatus is operable to receive data transfer by any suitable transfer medium in order to upgrade and/or change the settings of the apparatus. For example, at least one of the detector(s), storage device, audio controller, and speaker(s) is operable to receive data transfer by any suitable transfer medium in order to upgrade and/or change the settings of the apparatus. Preferably, at least one of the storage device and/or the audio controller are operably to receive data transfer by any suitable transfer medium in order to alter the audio output of the apparatus.

The phrase "suitable transport mediums" is used herein to include, but is not limited to, one or more transport medium selected from: IR signalling, visible light signalling, ultra sonic signalling, and radio frequency signalling, for example Bluetooth, Zigbee, powerG, Wireless X10, and/or WirelessHART. Wired connections between parts of the apparatus are also possible as will be appreciated.

The at least one detector preferably comprises at least one passive infra-red sensor, and in which the predetermined region is defined by the detection arc of the infra-red sensor(s).

The at least one detector preferably comprises at least one contact sensor located adjacent at least one point of entry to the predetermined region.

The or each contact sensor is preferably arranged to include two contact portions mountable on opposed surfaces of a door and an adjacent door frame located adjacent a point of entry to the predetermined region.

The storage device may comprise a Universal Serial Bus (USB) port for connection to a USB device comprising the audio output comprising at least one pre-recorded audio message.

The apparatus may further comprise a visual indicator providing a visual indication of the status of the apparatus.

The visual indicator may comprise an LED indicator.

The apparatus may be operable to communicate using any suitable transfer medium with a security system for the building comprising the predetermined region. The apparatus is preferably operable to send a signal using any suitable transfer medium to the security system to make the security system aware of the presence of a user within the predetermined region. The apparatus is preferably operable to send identification data using any suitable transfer medium to the security system to make the security system aware of the identification of the user within the predetermined region. The apparatus is preferably operable to receive one or more communication signals using any suitable transfer medium from the security system in order to communicate with the user, for example to provide further audio instructional outputs to the user in the case of an emergency.

According to a second aspect, the present invention provides method of providing directional and/or instructional audio messages to a user using an apparatus as herein described comprising detecting a user within and/or entering the predetermined region using the at least one detector, and controlling the audio output to the at least one speaker in order to provide at least one audio message from the storage device.

The method may further comprise storing at least one audio directional and/or instructional message on the storage device.

The method may further comprise identifying the user by detecting one or more identification signals, for example radiofrequency identification signals, emitted by a portable item carried by a user within the predetermined region.

The method may further comprise inserting a USB device comprising at least one audio directional and/or instruction message into a USB port of the storage device.

The method may further comprise receiving data transfer by any suitable transfer medium in order to upgrade and/or change the settings of the apparatus.

According to an aspect, there is provided a directional guidance system, comprising an apparatus as provided herein, and a portable device operable to communicate with the apparatus using a wireless link and receive data from the apparatus representing the at least one pre-recorded directional and/or instructional audio message. The portable device can be user equipment (UE) such as a mobile telephone or other such portable device with wireless, such as radio frequency, communication capabilities for example. The apparatus can include a short range radio frequency module and/or can be communicatively linked to a near field communication target device, either of which is operable to transmit data to the portable device. The device can include a short range radio frequency module and/or a near field communication module, and can receive data representing the at least one pre-recorded directional and/or instructional audio message using the short range radio frequency module and/or the near field communication module.

According to an aspect, there is provided a computer program product, comprising a computer usable medium having computer readable program code embodied therein, said computer readable program code adapted to be executed to implement a method for providing directional and/or instructional audio messages to a user as provided herein.

### BRIEF DESCRIPTION OF THE DRAWINGS

An embodiment of the present invention will now be described, by way of example only, and with reference to the accompanying drawings, in which:
Figure 1 is a schematic illustration of a directional guidance apparatus according to a first embodiment of the present invention;
Figure 2 is a schematic illustration of the apparatus of Figure 1 mounted to a wall;
Figure 3 is a schematic illustration of the apparatus of Figure 1 mounted to a ceiling;
Figure 4 is a schematic illustration of a wall mounted directional guidance apparatus according to a second embodiment of the present invention;
Figure 5 is a schematic representation of a system according to an example;
Figure 6 is a schematic representation of a system according to an example; and
Figure 7 is a schematic representation of a system according to an example.

### DETAILED DESCRIPTION

Example embodiments are described below in sufficient detail to enable those of ordinary skill in the art to embody and implement the systems and processes herein described. It is important to understand that embodiments can be provided in many alternate forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

The terminology used herein to describe embodiments is not intended to limit the scope. The articles "a," "an," and "the" are singular in that they have a single referent, however the use of the singular form in the present document should not preclude the presence of more than one referent. In other words, elements referred to in the singular can number one or more, unless the context clearly indicates otherwise. It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

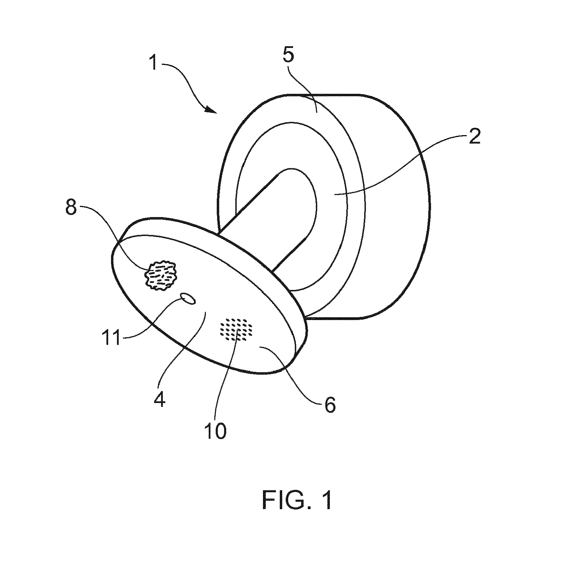

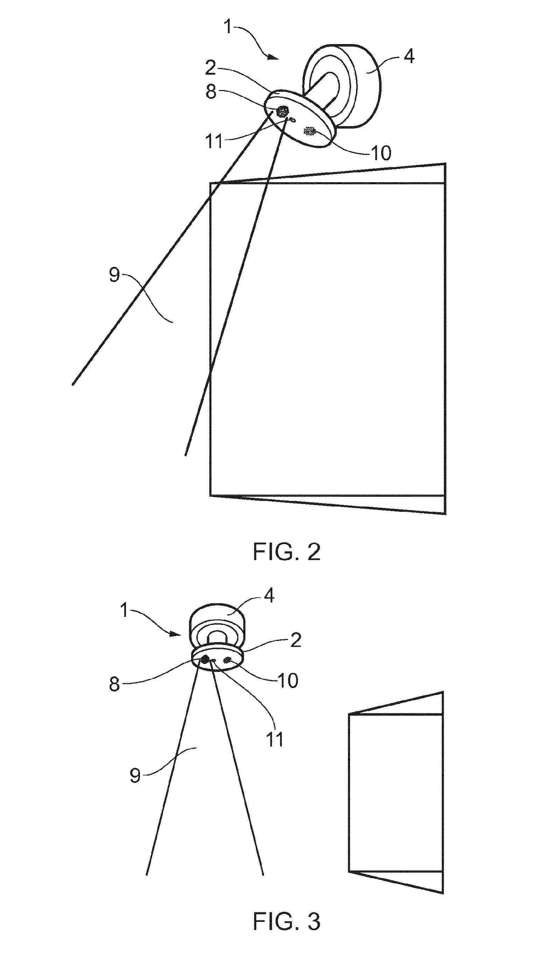

Figures 1 to 4 illustrate embodiments of the present invention placed within a bathroom/toilet area, such as for example an accessible toilet. With reference to Figures 1 to 4, the directional guidance apparatus 1 comprises a mountable portion 2 and a detector portion 4. The mountable portion 2 defines a mounting surface 5 for mounting on an adjacent surface, such as for example a wall (as shown in Figures 1 and 3) or on a ceiling (as shown in Figure 2). The mountable portion 2 may be secured to the adjacent surface by any suitable fixing means, such as for example by screws.

The detector portion 4 extends from the mountable portion 2. It is however to be understood that the detector portion 4 and mountable portion 2 may be provided as a unitary item. Alternatively, the detector portion may be releasably secured to the mountable portion aiding repair and replacement of the apparatus.

The apparatus 1 comprises a detector 8 which is capable of detecting the presence of a user entering and/or within a predetermined region, in this case a bathroom/toilet area such as for example an accessible toilet.

As shown in Figures 1 to 3, the detector portion 4 comprises a detector surface 6 comprising a passive infrared detector 8. The passive infrared detector 8 provides a detection arc 9 (as shown in the Figures) extending from the detector 8 towards the floor of the room. The detection arc 9 defines the pre-determined region which is monitored by the detector 8.

As shown in Figure 1, when wall mounted, the detector portion 4 defines a surface 6 which extends at an angle to the mounting surface 5 of the apparatus 1. The detector portion 4 is arranged such that the detection arc 9 provided by the detector 8 extends at an obtuse angle to the longitudinal axis of the mountable portion 2.

As shown in Figure 2, when ceiling mounted, the detector portion 4 of the apparatus 1 defines a surface 7 which extends substantially parallel to the mounting surface 5 of the apparatus 1. The detector portion 4 is arranged such that the detection arc 9 provided by the detector 8 extends substantially parallel to the longitudinal axis of the mountable portion 2.

Although the Figures show the detector portion 4 comprising a single detector it is to be understood that the apparatus may include any suitable number and/or types of detectors, in any suitable locations, depending on the particular requirements for the predetermined region. The location of the apparatus I within the room and/or orientation of the detector relative to the mountable portion 2 may be selected and/or adjusted such that the detection arc(s) 9 is provided in the preferred location within the room.

The detector portion 4 may for example be rotatably fixed relative to the mountable portion 2. Alternatively, the detector portion 4 may be rotatable relative to the mountable portion 2 so that the detection arc provided by the or each detector(s) can be manoeuvred so as to provide the detection arc at the desired location so as to optimise the detection of a user within the room.

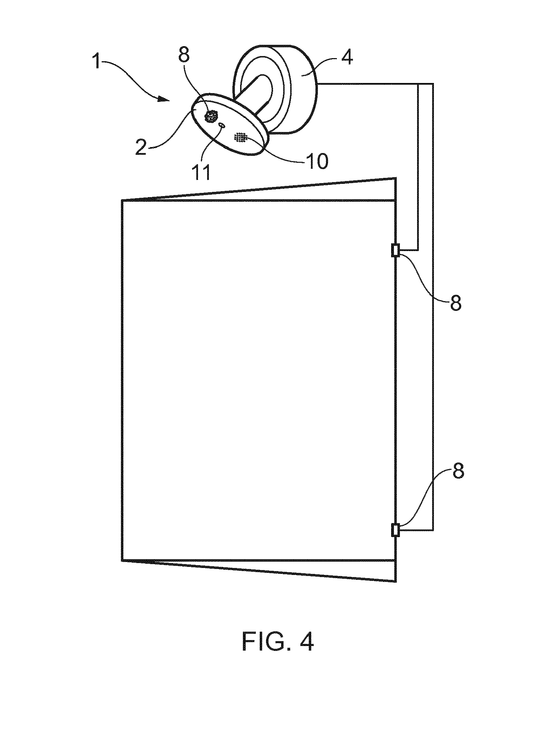

As shown in Figure 4, the apparatus comprises a contact detector 8 comprising two contact portions which are located on opposing surfaces of the door to the bathroom/toilet area (such as for example an accessible toilet) and the adjacent door frame. The contact portions provide an electrical connection extending between the door and door frame when the door is closed. This electrical connection is broken when a user enters or leaves the bathroom/toilet area (such as for example an accessible toilet).

The apparatus I further comprises a LED display 11 operable to provide a visual indication of the status of the unit.

The apparatus I further comprises a storage device which is operable to store an audio output comprising at least one pre-recorded directional and/or instructional audio message. The selection of stored messages may be selected and/or varied to suit the particular requirements of the room. In the Examples shown in the Figures, the apparatus I is used to provide guidance to a visually impaired user as to the location of items within an unfamiliar toilet/bathroom area, such as for example an unfamiliar accessible toilet.

The list of audio guidance and/or instructional messages may therefore, for example, include one or more of the following:
instructions as to the location and/or type of light switch within the area;
instructions as to the location and/or type of door lock;
instructions as to the location of the toilet within the area;
instructions as to the location of the toilet tissue;
instructions as to the height of the cistern;
instructions as to the location and manner of operation of the flush;
instructions as to the location of an Emergency Pull Cord;
instructions as to the location of the sanitary box;
instructions as to the location and/or manner of operation of the baby change facilities;
instructions as to the location and/or type of wash basin within the area;
instructions as to the location and/or manner of operation of the soap dispenser;
instructions as to the location, number of and/or manner of operation of the taps;
instructions as to the location and/or manner of operation of the hand drier;
guidance/information about taps e.g. are there two taps; a single tap, lever taps or press button operation; hot tap on left/right etc;
instructions as to the location of the exit from the area; and/or
instructions as to how to open the door.

The audio output may for example be stored on a USB interface. The apparatus 1 may comprise a USB port for receiving the audio output from a USB interface. The audio output may be communicated to the speaker by any suitable means, such as for example by wireless communication or radio frequency communication.

The detector portion 4 further includes a speaker 10. Although the figures illustrate the speaker as being located on the detector portion 4, it is to be understood that the speaker may be provided at any suitable location on the apparatus I, such as for example the mountable portion, and may in some embodiments be provided as a separate component in communication with the detector and the storage device.

It is also to be understood that the apparatus may include any suitable number of speakers on or in communication with the detector and storage device depending on the requirements of the room. For example, the apparatus may include a plurality of spaced apart speakers. In one embodiment, the speakers may be arranged such that each speaker is located on a separate wall or section of ceiling of the room, or adjacent individual pieces of apparatus.

The apparatus I further comprises an audio controller operable to control the audio output to the or each speaker on detection of a user within the predetermined region in order to provide at least one audio message from the storage device. The audio controller is preferably arranged to provide a sequence of audio outputs in a sequence which corresponds to normal user usage of the bathroom/toilet area, such as for example an accessible toilet.

In one embodiment, the apparatus 1 may include a plurality of speakers. Each speaker located on a wall or ceiling portion adjacent to an item of sanitary ware, such as for example a toilet, a basin, or a hand dryer, and the door to the room. The audio controller may be operable to control which audio output is delivered to each speaker, and the timing of each audio output. The audio controller may therefore be arranged to deliver each particular audio output to the speaker located adjacent the relevant article of the audio output in order to provide improved directional guidance to the user.

In use, the user enters the bathroom/toilet area, such as for example an accessible toilet. A detector 8, either the passive infrared detector of the embodiments of Figures 1 and 2 or the contact sensor of Figure 3, detects the presence of the user within the predetermined region (such as for example within the detection arc). The audio controller operates the storage device and the speaker 10 to provide at least one, preferably a sequence of, audio outputs to the user to provide directional and/or instructional guidance to the user. Preferably, the audio controller controls the timing between each audio output so as to provide instructions at relevant timings for normal use of the bathroom/toilet facilities. In the case where the apparatus comprises multiple speakers, the audio controller controls which audio output is delivered to which speaker in order to provide further improved directional guidance to the user.

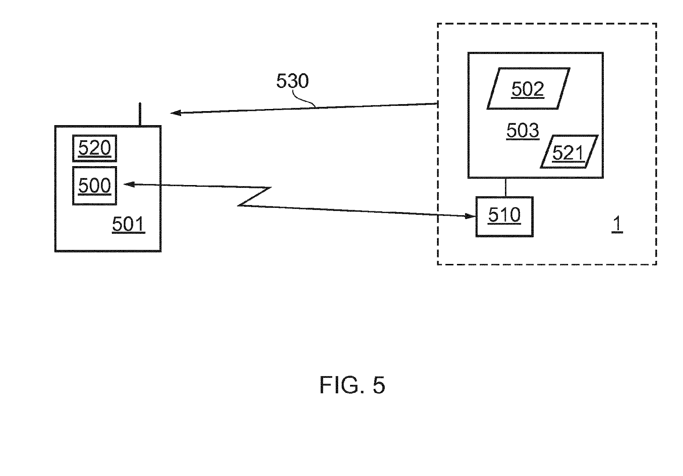

Figure 5 is a schematic representation of a system according to an example. In the example of figure 5, user equipment (UE) 501, such as a mobile telephone or other suitable portable device for example, can communicate using a short range radio frequency protocol, such as Bluetooth and so on. An apparatus, I, such as described with reference to figures 1-4 for example, includes a short range radio frequency protocol module 510, which enables the apparatus I to wirelessly send and receive data using a short range radio frequency protocol. Accordingly, UE 501 and apparatus 1 can communicate when UE 501 is within range of the module 500 of the apparatus 1. A short range radio frequency protocol module 501 is provided at UE 501.

In the example of figure 5, UE 501 can be discoverable. That is, the apparatus 1 can search, connect and transfer data with UE 501. Thereafter, data 502 identifying the UE 501 can be stored in a storage device 503 of apparatus 1. In subsequent communications, UE 501 may not be in a discoverable mode of operation. However, the apparatus 1 can identify the UE 501 using the previously stored data, as is typical for such ad-hoc short range communications. The UE 501 can transmit data 521 to apparatus I relating to user preferences or parameters. For example, the UE 501 can be programmed by a user to include preferences 520 for instructions to be provided by an apparatus, such as apparatus 1. The preferences can indicate, for example, whether the user may require a full set of instructions or only partial instruction, such as in the event that the user is partially sighted for example. For example, the user may only require instructions about whereabouts in the room in question a toilet is located. When device 501 initially communicates with apparatus I, such as when in a discoverable phase for example, the UE 501 can prompt a user to provide permission to transmit data 521 representing preferences 520 to the apparatus I, which can be transmitted if permission is given by a user.

In an example, data 502 can be used to authenticate UE 501. In the case that UE 501 is authenticated and trusted for example, apparatus 1 can proceed to provide instructions upon determining the presence of a user (and thus UE 501). The apparatus may not accept data 521 in the event that the UE 501 is not authenticated or not trusted.

Also, if the UE 501 is not authenticated or trusted, instructions may not be provided. For example, an owner of a facility may wish to constrain the number of users of a particular facility to selected users. As such, certain user's devices may be trusted devices. Detected presence of such a device may therefore allow the user to receive instructions from an apparatus of the facility.

For example, if the presence of a user is detected by apparatus I, the UE 501 can be queried by apparatus 1 by issuing a challenge to the UE 501 based on data 502 or a portion of data 502. For example, apparatus 1 can send a unique challenge value x to the UE 501. UE 501 can generate a unique challenge value y. The UE 501 computes r = hash(y + x + secret). The UE 501 can then transmit r and y to the apparatus 1 using the short range wireless link between the devices.

Apparatus 1 can calculate the expected value of r and ensure the UE 501 responded correctly. Optionally, apparatus 1 can compute ar = hash(x + y + secret), and transmit ar to the UE 501. UE 501 can calculate the expected value of ar and ensures the apparatus responded correctly. This can provide security for the UE, so that it knows it is communicating with a trusted entity, as well as security for the apparatus.

Here, x is the apparatus generated challenge, y is the UE generated challenge, r is the UE response, and ar is the apparatus response. Alternative procedures may be used, as will be apparent to those skilled in the art, in order to authenticate the UE 501 (and/or apparatus 1) can ensure the entities are trusted.

In the event that it is determined that the UE or apparatus is not trusted (for example, the generated responses are not as expected), communication links between the devices can be severed.

In an example, an apparatus may communicate with a device, such as a UE as described with reference to figure 5, in order to provide instructions to a user via the device itself, or in combination with the apparatus. For example, an apparatus may not include a speaker or other output device, but instead may provide data to a UE in order for the UE to provide instructions for a user. Providing data for a UE device may alternatively be in addition to instructions provided using an audio output device of the apparatus.

For example, audible instructions may be provided using a speaker of the UE. In addition, a display of the UE may be used to provide information for a user, such as directional or proximity information representing the direction to an element within a facility and/or the proximity of the user to the element. Other feedback devices of the UE may be used, such as vibration or other tactile and/or visual alerts or indications.

That is, in an example, data representing the provision and/or the position of certain elements of a facility can be provided to a UE, which may be a trusted and/or authenticated device as noted above. The device can use the received data in order to provide instructions and/or indications for a user, such as audible instructions and/or indications and/or visual instructions and/or indications and/or other audible or visual instructions and/or indications such as vibrations, visual displays of proximity and so on. A UE may include a GPS module for example, which will enable the UE to determine its present location. Based on a set of coordinates relating to an element in a facility, the UE can therefore determine proximity to the element, and include a directional element relating to the direction in which the UE is moving for example. Accelerometers and the like can also be used in order to provide an indication of direction or orientation of the UE, which can be used with position information to provide instructions to a user with a higher degree of accuracy.

Accordingly, a UE 501 can be discovered by apparatus 1 in an initial discovery phase in which the UE is paired with the apparatus I, which can store data 502 relating to the UE 501, such as a device identification and other data representing the device and/or the user. The UE 501 can be authenticated in subsequent encounters with apparatus 1 when its presence is detected in order to ensure that the device is a trusted device. Preference data 521 can be provided to the apparatus I, and can be updated at periodic intervals, such as every time the UE and the apparatus connect to one another and/or in the event that updated preferences 520 are available, or by a user selecting or causing the preference data to be transmitted/updated.

The UE 501 can be used in addition to or in place of apparatus I to provide instructions to a user. Data 530 representing instructions to be given can be transmitted from apparatus I to UE 501. The instructions can the same as the instructions that would otherwise have been provided by the apparatus I, or may be augmented or tailored as described. As such, it is possible to provide augmented and/or tailored instructions for users with certain devices that are able to communicate with the apparatus using one of the mechanisms described above. Augmented instructions can provide additional feedback for a user relating to their position, orientation and direction of travel for example, with respect to the position of certain elements of a facility. The instructions may also be tailored for the individual based on user preferences or parameters. In an example, the data transmitted to a device can be used to provide instructions for a user in the absence of any other instructions from the apparatus. Accordingly, the apparatus may only function with such devices, and may not be provided with any other output devices, such as speakers for example.

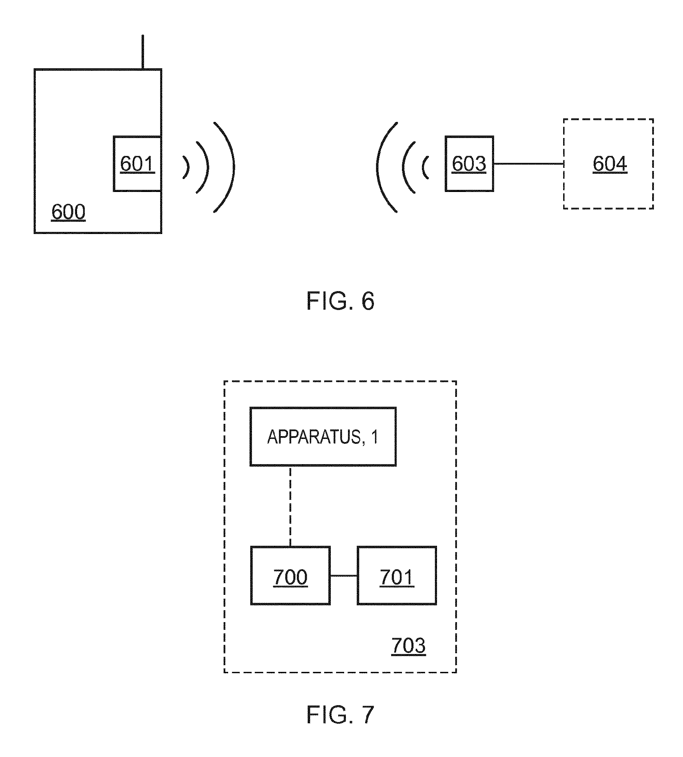

Figure 6 is a schematic representation of a system according to an example. Similarly to figure 5, a device, such as UE, which may be a mobile telephone for example, is provided. The device 600 can include a near field communications device 601, which can be used by the device 600 for short-range wireless communication, typically at a distance of 10 cm or less, with a target 603, which can be a passive device, such as an RF tag for example. Accordingly, device 600 can read and/or write data to the target 603.

The target 603 can include data relating to a facility 604. For example, the target 603 may be placed outside an accessible toilet, and can include data representing the facilities on offer and the position of such facilities. Several possibilities can be provided by such a target device. For example, an apparatus I can be triggered when the presence of a user is determined. The apparatus can determine the presence of an NFC capable device using a short range wireless protocol as described above, for example by querying the device for an indication of device capabilities, which can be provided automatically or by manual intervention of a user. If a user has a device that is NFC enabled, the apparatus 1 can direct the user using audible and/or visual instructions to the target 603. The instructions may be provided by the UE itself. For example, the UE can receive data representing instructions to the location of the target. The user can then bring their device into proximity with the target 603, can data for the facility can be transferred to the device. As such the data for the facility can be regularly updated by updating the target device, which can be performed by a building administrator and so on for example. The target may be provided on a movable platform such as sign, such that the platform and target can be moved as desired. For example, the target can be moved to another location in the event that a facility is moved or is no longer in use.

As noted above, the target 603 can include data relating to the device 600. As such, when brought into proximity with the target 603, it can query the device 600 and perform authentication, as noted above with reference to figure 5. In the event of authentication failure, the target 603 may not transfer any further data to the device for example.

Upon completion of data transfer, the target 603, which may be coupled to the apparatus 1 wirelessly or using a wired connection, can transmit a signal to the apparatus I to indicate that data transfer is complete. The apparatus 1 and/or the target 603 can then trigger the device to provide instructions for the user as described above. The instructions provide can be in addition to, or in place of, instructions provided by apparatus 1.

As such, it is possible to provide augmented and/or tailored instructions for users with certain devices that are able to communicate with the apparatus using one of the mechanisms described above. In the event that a user does not have a device as described, the apparatus may provide instructions as described with reference to figures 1-4. That is, augmented instructions can provide additional feedback for a user relating to their position, orientation and direction of travel for example, with respect to the position of certain elements of a facility. The instructions may also be tailored for the individual based on user preferences or parameters. In an example, the data transmitted to a device can be used to provide instructions for a user in the absence of any other instructions from the apparatus. Accordingly, the apparatus may only function with such devices, and may not be provided with any other output devices, such as speakers for example.

Figure 7 is a schematic representation of a system according to an example. In the example of figure 7, which is compatible with any of the apparatus or systems as described above, apparatus I is linked to a system of the building in which the facility in question is situated. The system 700 of the building 703 can be a security monitoring system for example, which is linked to a central security system 701. Accordingly, building alerts can be provided by apparatus 1 in response to an instruction from system 701. For example, apparatus 1 can provide or interrupt instructions being given to provide instructions relating to a security announcement. For example, the apparatus can be used to provide an announcement for a user that is experiencing difficulties and requires assistance. In this connection, an alarm 703, which may be activated by a user, can trigger the system 701 to indicate that a user requires assistance. The system 701 can then send a signal for apparatus I to enable instructions and/or a message from security personnel to be relayed to the user, such as a message indicating that help is on the way for example. Other messages can be provided, which can override any instructions currently being provided. For example, a fire alert can be provided, along with associated instructions directing the user to the nearest exit.

Although a device with reference to figures 5-7 is described with reference to a UE, it will be appreciated that the device may be a simple device that only has the function of communicating with an apparatus as described, using the processes of either (or both) as described with reference to figures 5 and 6 for example. As such, a device according to an example can be a low cost and/or low complexity device.

Although the Figures illustrate the apparatus located within a bathroom/toilet area, such as for example an accessible toilet, it is to be understood that the apparatus can be used in any suitable area for providing directional guidance to a user, such as for example to a user with partial, severe and/or total sight loss, to locate and/or operate any suitable equipment.

The present inventions may be embodied in other specific apparatus and/or methods. The described embodiments are to be considered in all respects as only illustrative and not restrictive. In particular, the scope of the invention is indicated by the appended claims rather than by the description and figures herein. All changes that come within the meaning and range of equivalency of the claims are to be embraced within their scope.

## Claims

1. A directional guidance apparatus, comprising:
at least one detector operable to detect a user within and/or entering a predetermined region;
a storage device operable to store an audio output comprising at least one pre-recorded directional and/or instructional audio message;
at least one speaker in communication with the audio output of the storage device; and
an audio controller operable to control the audio output to the speaker on detection of a user within the predetermined region in order to provide at least one audio message.

2. An apparatus as claimed in claim 1, in which the apparatus comprises a main body unit comprising a mountable portion comprising a mounting surface for mounting the main body unit on a wall or ceiling, and a detector portion comprising a detector surface comprising at least one detector.

3. An apparatus as claimed in claim 2, in which the detector portion and the mountable portion are provided within an integral main body unit.

4. An apparatus as claimed in claim 3, in which the plane defined by the detector surface extends substantially parallel to the mounting surface of the mounting portion.

5. An apparatus as claimed in claim 3, in which the plane defined by the detector surface extends at an angle to mounting surface of the mounting portion.

6. An apparatus as claimed in claim 5, in which the detector portion is moveable relative to the mountable portion so as to adjust the angle of the detector surface relative to the mounting surface.

7. An apparatus as claimed in any preceding claim, further comprising a portable unit in communication with at least one of the detector(s), storage device, speaker(s), and audio controller.

8. An apparatus as claimed in claim 7, in which the portable unit comprises the speaker(s), and in which the portable unit comprises a module operable to receive signals from the audio controller to control the audio output of the speaker.

9. An apparatus as claimed in claim 7, in which the portable unit is in communication with the detector (s).

10. An apparatus as claimed in any one of claims 1 to 9, in which the at least one detector comprises at least one passive infra-red sensor, and in which the predetermined region is defined by the detection arc of the infra-red sensor(s).

11. An apparatus as claimed in any preceding claim, in which the at least one detector comprises a contact sensor located adjacent a point of entry to the predetermined region.

12. An apparatus as claimed in claim 11, in which the contact sensor is arranged to include two contact portions mountable on opposed surfaces of a door and an adjacent door frame located adjacent a point of entry to the predetermined region.

13. An apparatus as claimed in any preceding claim, in which the storage device comprises a Universal Serial Bus (USB) port for connection to a USB device comprising the audio output comprising at least one pre-recorded audio message.

14. An apparatus as claimed in any preceding claim, further comprising a visual indicator providing a visual indication of the status of the apparatus.

15. A method of providing directional and/or instructional audio messages to a user using an apparatus as claimed in any one of claims I to 15, comprising:
detecting a user within and/or entering the predetermined region using the at least one detector; and
controlling the audio output to the at least one speaker in order to provide at least one audio message from the storage device.

16. A method as claimed in claim 16, further comprising storing at least one audio directional and/or instructional message on the storage device.

17. A method as claimed in claim 17, further comprising inserting a USB device comprising at least one audio directional and/or instruction message into a USB port of the storage device.

18. A directional guidance system, comprising:
an apparatus as claimed in any of claims I to 15; and
a portable device operable to communicate with the apparatus using a wireless link and receive data from the apparatus representing the at least one pre-recorded directional and/or instructional audio message.

19. A directional guidance system as claimed in claim 19, wherein the apparatus includes a short range radio frequency module and/or is communicatively linked to a near field communication target device, either of which is operable to transmit data to the portable device.

20. A portable device for use with an apparatus as claimed in any of claims I to 15, the device including a short range radio frequency module and/or a near field communication module, wherein the device is operable to receive data representing the at least one pre-recorded directional and/or instructional audio message using the short range radio frequency module and/or the near field communication module.

21. A computer program product, comprising a computer usable medium having computer readable program code embodied therein, said computer readable program code adapted to be executed to implement a method for providing directional and/or instructional audio messages to a user as claimed in any of claims 16 to 18.
